# FASCICULE DE BREVET EUROPEEN

(11) **EP 2 473 133 B1**
(45) Date de publication et mention de la délivrance du brevet: **09.04.2014**
(21) Numéro de dépôt: 10763813.2
(22) Date de dépôt: 06.09.2010
(51) Int. Cl.: A61F 2/00, A61L 31/10, A61L 31/14, D04B 21/12

(54) **TISSU AVEC PICOTS REVETUS D'UN MATERIAU HYDROSOLUBLE**
STOFF MIT ÖSEN MIT BESCHICHTUNG AUS EINEM WASSERLÖSLICHEN MATERIAL
FABRIC COMPRISING PICOTS COATED WITH A HYDROSOLUBLE MATERIAL

(30) Priorité: 04.09.2009 FR 0956038
(43) Date de publication de la demande: 11.07.2012
(73) Titulaire: Sofradim Production, 01600 Trévoux (FR)
(72) Inventeur: MONTANARI, Suzelei, F-01600 Trevoux (FR); REY, Pearl, F-38500 Saint Nicolas de Macherin (FR)
(74) Mandataire: Verriest, Philippe
(86) Numéro de dépôt international: PCT/FR2010/051843
(87) Numéro de publication internationale: WO 2011/027087

(56) Documents cités:
- WO-A1-01/81667
- WO-A2-2009/039429
- FR-A1- 2 924 330

## Description

La présente invention se rapporte à un tissu prothétique muni de picots recouverts d'un revêtement hydrosoluble. Un tel tissu est particulièrement utilisable pour la réalisation de prothèses de renfort de paroi destinées à être introduites chez un patient par coelioscopie.

Les prothèses de renfort de paroi, par exemple abdominale, sont largement utilisées dans le domaine chirurgical. Ces prothèses sont destinées à traiter des hernies en venant combler, temporairement ou définitivement, un défaut tissulaire. Ces prothèses sont généralement réalisées en tissu prothétique biocompatible et peuvent avoir plusieurs formes, rectangulaire, ronde, ovale, selon la structure anatomique à laquelle elles doivent s'adapter. Certaines de ces prothèses sont réalisées à partir de fils entièrement biorésorbables et sont destinées à disparaître après avoir assuré leur fonction de renfort le temps que la colonisation cellulaire s'effectue et que la réhabitation tissulaire prenne le relais. D'autres comprennent des fils non biorésorbables et sont destinées à rester définitivement dans le corps du patient.

Certaines de ces prothèses sont réalisées à partir d'un arrangement de fils, tricot, tissage ou encore non tissé, comprenant des poils à picots saillant à l'extérieur d'une face de la prothèse : ces picots constituent des crochets capables de venir se fixer soit dans un autre tissu prothétique, appartenant à la même prothèse ou non, soit directement dans les tissus biologiques, comme par exemple la paroi abdominale. Certaines prothèses peuvent également comprendre des picots sur chacune de leurs deux faces.

Par ailleurs, dans le souci de minimiser les traumatismes subséquents à toute intervention chirurgicale, on opère de plus en plus souvent les patients par voie coelioscopique lorsque le type d'intervention pratiquée le permet. La voie coelioscopique ne nécessite que de très petites incisions, au travers desquelles on fait passer un trocart, au sein duquel on achemine la prothèse jusqu'au site d'implantation. On évite ainsi une chirurgie ouverte et le patient peut quitter l'hôpital rapidement. La voie coelioscopique est particulièrement prisée dans les interventions chirurgicales pratiquées au niveau de l'abdomen, comme par exemple le traitement des hernies.

Toutefois, les trocarts utilisés en voie coelioscopique présentent en général un diamètre calibré relativement faible, pouvant varier par exemple de 5 à 15 mm, afin de réduire au maximum la taille de l'incision pratiquée. La prothèse doit donc être acheminée au sein d'un conduit de diamètre réduit et elle doit ensuite être déployée sur le site d'implantation.

Pour effectuer cette étape, la prothèse est généralement enroulée sur elle-même afin de la faire glisser dans le conduit du trocart. Toutefois, lorsque le tissu prothétique formant la prothèse comprend des picots sur une face, il peut arriver que ces picots, du fait qu'ils ne sont pas protégés, s'entremêlent dans le tissu ou soient endommagés lors de l'enroulage de la prothèse ou lors de son acheminement à travers le trocart jusqu'au site d'implantation.

Ainsi, il subsiste le besoin d'un tissu prothétique comprenant des poils à picots, utilisable pour la fabrication de prothèses, comme par exemple des renforts de paroi abdominale, capable d'être acheminé au sein d'un conduit tel que celui d'un trocart, sans endommager les picots, et capable de se déployer totalement une fois atteint le site d'implantation dans le corps du patient.

La présente invention vise à remédier à un tel besoin.

Un premier aspect de la présente invention concerne un tissu prothétique comprenant un arrangement de fils définissant au moins deux faces pour ledit tissu, ledit tissu comprenant sur au moins une de ses faces un ou plusieurs picots saillant vers l'extérieur par rapport à ladite face, caractérisé en ce que lesdits picots sont recouverts d'un revêtement en matériau biocompatible hydrosoluble.

Par matériau hydrosoluble, on entend au sens de la présente demande un matériau capable de se dissoudre dans une composition aqueuse telle que de l'eau ou les fluides biologiques, par exemple à température ambiante, soit environ à une température d'environ 20 à 25°C, ou à toute température supérieure et en particulier à la température du corps humain, autrement dit à une température d'environ 37°C.

De préférence, le matériau hydrosoluble du tissu selon l'invention est sous forme figée ou solide à une température inférieure ou égale à 35°C, lorsqu'il n'est pas mis au contact d'une composition aqueuse.

Ainsi, lorsque le tissu selon l'invention est mis en contact avec de l'eau ou des fluides biologiques, en particulier à la température du corps humain soit environ 37°C sur le site d'implantation, le matériau recouvrant les picots se solubilise peu à peu et ainsi se détache des picots. Le temps nécessaire à la solubilisation de l'ensemble du matériau recouvrant les picots permet de déployer la prothèse de façon aisée comme il sera expliqué plus en détails ci-dessous. Une fois que les picots ne sont plus recouverts, ils peuvent à nouveau jouer leur rôle de fixation, soit à un autre tissu prothétique, soit au sein d'un tissu biologique, comme par exemple la paroi abdominale.

En général, le matériau recouvrant les picots, du fait de sa nature hydrosoluble, présente une surface lisse à une température inférieure ou égale à 25°C : ainsi, lorsqu'un picot recouvert de matériau hydrosoluble entre en contact avec un autre picot recouvert de matériau hydrosoluble, ils glissent l'un sur l'autre et ne s'opposent pas de résistance. De fait, lorsqu'on enroule la prothèse sur elle-même afin de la faire glisser dans un trocart, les picots s'entremêlent les uns dans les autres, mais pas de façon définitive : les picots ne s'accrochent pas les uns aux autres, ils glissent les uns sur les autres et ils se séparent les uns des autres aisément dès que la prothèse est libérée des parois du trocart dans le site d'implantation : la prothèse se déploie alors facilement avant que l'ensemble du matériau hydrosoluble recouvrant les picots ne soit totalement solubilisé.

Par ailleurs, ce nécessaire temps de solubilisation, de l'ordre de quelques secondes à quelques minutes, permet également au chirurgien de positionner la prothèse, en la déplaçant si nécessaire, et ce de façon aisée puisque, le matériau hydrosoluble n'étant pas encore totalement solubilisé, les picots glissent vis-à-vis des tissus environnants et ne fixent pas encore la prothèse comme ils le feront une fois qu'ils seront totalement dépourvus de revêtement en matériau hydrosoluble.

Dans une forme de réalisation de l'invention, ledit matériau hydrosoluble est biodégradable.

Par biodégradable, on entend, au sens de la présente demande, un matériau apte à être résorbé, absorbé, et/ou dégradé par les tissus ou lessivé du site d'implantation et disparaissant in vivo après un certain temps, qui peut varier, par exemple, de quelques heures à quelques mois, suivant la nature chimique du matériau.

Dans une forme de réalisation, ledit matériau hydrosoluble est choisi parmi les polyéthylène glycols (PEG), les acétates de polyvinyle (PVA), la gélatine, l'acide polyglucoronique, l'acide hyaluronique, la carboxyméthycellulose, les éthers de cellulose, les chitosan et leurs mélanges.

Par exemple, ledit matériau hydrosoluble est un polyéthylène glycol de masse molaire inférieure ou égale à 40 000 Da, de préférence inférieure ou égale à 20 000 Da. Par exemple, ladite masse molaire peut varier de 1000 à 20 000 Da. Les polyéthylène glycols présentant de telles masses molaires sont particulièrement biodégradables.

Dans une forme de réalisation, ledit matériau hydrosoluble comprend au moins un polyéthylène glycol de masse molaire environ 1000 Da. Un tel polyéthylène glycol permet une solubilisation améliorée.

Dans une forme de réalisation, ledit matériau hydrosoluble comprend en outre un polyéthylène glycol de masse molaire différente de 1000 Da.

Les chitosan convenant pour le matériau hydrosoluble de la présente invention sont les chitosan ou dérivés de chitosan hydrosolubles, comme par exemple les chitosan partiellement N-acétylés décrits dans la publication « Water-solubility of partially N-acetylated chitosans as a function of pH : effect of chemical composition and depolymerisation », Kjell M .Vårum, Mette H. Ottøy & Olav Smidsrød, Carbohydrate Polymers 25 (1994) 65-70.

Un autre aspect de la présente invention porte sur un procédé pour recouvrir un tissu prothétique comprenant un arrangement de fils définissant au moins deux faces pour ledit tissu, ledit tissu comprenant sur au moins une de ses faces un ou plusieurs picots saillant vers l'extérieur par rapport à ladite face, caractérisé en ce qu'il comprend les étapes suivantes :
- a°) on prépare une composition comprenant au moins un matériau biocompatible hydrosoluble à l'état liquide,
- b°) on applique une couche de ladite composition sur lesdits picots.

La composition comprenant le matériau biocompatible hydrosoluble à l'état liquide peut être sous forme liquide, visqueuse ou encore pâteuse. Par exemple, la composition présente une consistance permettant son étalement sur les picots, par exemple à l'aide d'un pinceau ou encore d'un rouleau, ou permettant l'immersion en son sein des picots. Dans une forme de mise en oeuvre du procédé selon l'invention, l'étape b°) est réalisée à l'aide d'un rouleau. Ainsi, la composition est uniformément répartie sur le rouleau puis sur les picots sur lesquels le rouleau est étalé.

Dans une forme de mise en oeuvre du procédé selon l'invention, ladite composition est obtenue par solubilisation dudit matériau hydrosoluble dans de l'eau, éventuellement en chauffant ladite composition à la température de fusion dudit matériau hydrosoluble.

Dans une telle forme de réalisation, une fois la couche de composition appliquée sur les picots, on laisse la composition sécher, et éventuellement refroidir, jusqu'à ce que la couche de matériau hydrosoluble revêtant les picots soit à l'état figé, c'est-à-dire solide.

Dans une autre forme de mise en oeuvre du procédé selon l'invention, ladite composition est obtenue par chauffage dudit matériau hydrosoluble seul, à une température supérieure à la température de fusion dudit matériau hydrosoluble. Dans une telle forme de réalisation, une fois la couche de composition appliquée sur les picots, on laisse la composition refroidir jusqu'à ce que la couche de matériau hydrosoluble revêtant les picots soit à l'état figé, c'est-à-dire solide.

Un autre aspect de la présente invention se rapporte à une prothèse pour le traitement de hernie, fabriquée à partir d'un tissu tel que décrit ci-dessus ou à partir d'un tissu obtenu par le procédé décrit ci-dessus.

La présente invention va maintenant être décrite plus en détails à l'aide de la description qui suit et des figures en annexe dans lesquelles :
- La Figure 1 est une vue schématique en coupe d'un tissu prothétique avec picots de l'art antérieur,
- La Figure 2 est une vue schématique en coupe d'un tissu prothétique selon l'invention,
- La Figure 3 est une vue schématique en coupe d'une autre forme de réalisation d'un tissu selon l'invention.

Selon la présente demande, on entend par tissu prothétique tout tissu obtenu par un arrangement ou un assemblage de fils, fibres, filaments et/ou multifilaments biocompatibles, tel que tricotage, tissage, tressage, non tissé. L'arrangement de fils du tissu selon l'invention définit au moins deux faces opposées. Le tissu prothétique selon l'invention comprend en outre des picots saillant d'au moins une de ces deux faces. Ces picots peuvent saillir de ladite face substantiellement perpendiculairement au plan de ladite face ou alternativement selon un ou plusieurs plans inclinés par rapport au plan de ladite face. Ces picots sont destinés à fonctionner comme des moyens de fixation, soit en s'entremêlant dans un ou plusieurs arrangements de fils, fibres, filaments et/ou multifilaments d'un autre tissu prothétique, soit en s'ancrant dans les tissus biologiques comme par exemple une paroi abdominale.

Les picots du tissu prothétique selon l'invention peuvent être formés à partir de fils, par exemple des fils monofilaments, thermofusibles, directement issus de l'arrangement de fils formant le tissu. De tels tissus et picots ainsi que leur procédé de fabrication sont par exemple décrits dans les demandes WO01/81667, DE 198 32 634 ou encore dans les brevets US 6, 596, 002, US 5, 254, 133.

Dans de tels cas, par exemple, les picots sont formés à partir de fils monofilaments en acide polylactique.

Alternativement, les picots du tissu prothétique selon l'invention peuvent être tout crochet réalisé en tout matériau biocompatible, solidaire de l'arrangement de fils formant ledit tissu, que ces crochets aient été incorporés audit tissu lors de la fabrication (tressage, tricotage tissage, etc...) dudit arrangement de fils ou aient été rapportés après.

Les fils, ou fibres ou filaments et/ou multifilaments formant l'arrangement de fils du tissu selon l'invention peuvent être réalisés en tout matériau biocompatible, biodégradable ou non. Ainsi, les matériaux biodégradables convenant pour les fils du tissu de la présente invention peuvent être choisis parmi l'acide poly(lactique) (PLA), l'acide poly(glycolique) (PGA), la cellulose oxydée, la polycaprolactone (PCL), la polydioxanone (PDO), le trimethylene carbonate (TMC), le polyvinyl alcool (PVA), les polyhydroxyalkanoates (PHAs), les polyamides, les polyethers, les copolymères de ceux-ci et les mélanges de ceux-ci. Les matériaux non biodégradables convenant pour les fils du tissu de la présente invention peuvent être choisis parmi le polyethylene terephthalate (PET), des polyamides, des aramides, le polytetrafluoroethylene expansé, le polyurethane, le polyvinylidene difluoride (PVDF), des polybutylesters, le PEEK (polyetherethercétone), des polyolefines (tel que le polyethylène ou le polypropylène), des alliages de cuivre, des alliages d'argent, du platine, des grades médicaux d'acier comme l'acier inoxydable de grade médical, des combinaisons de ceux-ci.

Dans une forme de réalisation de l'invention, le tissu prothétique peut être pourvu de picots sur ses deux faces.

Les picots du tissu prothétique selon l'invention sont recouverts d'un matériau biocompatible hydrosoluble.

Par matériau hydrosoluble, on entend au sens de la présente demande un matériau capable de se dissoudre dans une composition aqueuse telle que de l'eau ou les fluides biologiques, par exemple à température ambiante, soit environ à une température d'environ 20 à 25°C, ou à toute température supérieure et en particulier à la température du corps humain, autrement dit à une température d'environ 37°C.

De préférence, le matériau hydrosoluble est sous forme solide ou figée à température ambiante et/ou à température de stockage du tissu selon l'invention, c'est-à-dire à une température environ inférieure ou égale à environ 35°C, lorsqu'il n'est pas mis au contact d'une composition aqueuse.

Dans une forme de réalisation de l'invention, ledit matériau hydrosoluble est biodégradable.

Par biodégradable, on entend, au sens de la présente demande, un matériau apte à être résorbé, absorbé, et/ou dégradé par les tissus ou lessivé du site d'implantation et disparaissant in vivo après un certain temps, qui peut varier, par exemple, de quelques heures à quelques mois, suivant la nature chimique du matériau.

Dans une forme de réalisation, ledit matériau hydrosoluble est choisi parmi les polyéthylène glycols (PEG), les acétates de polyvinyle (PVA), la gélatine, l'acide polyglucoronique, l'acide hyaluronique, la carboxyméthycellulose, les éthers de cellulose, les chitosan et leurs mélanges.

Par exemple, ledit matériau hydrosoluble est un polyéthylène glycol de masse molaire inférieure ou égale à à 40 000 Da, de préférence inférieure ou égale à 20 000 Da. Ladite masse molaire peut varier de 1000 à 20 000 Da. Les polyéthylène glycols présentant de telles masses molaires sont particulièrement biodégradables.

Dans une forme de réalisation, ledit matériau hydrosoluble comprend au moins un polyéthylène glycol de masse molaire environ 1000 Da. Un tel polyéthylène glycol permet une solubilisation améliorée.

Dans une forme de réalisation, ledit matériau hydrosoluble comprend en outre un polyéthylène glycol de masse molaire différente de 1000 Da.

Pour préparer un tissu selon l'invention, on dispose généralement d'un tissu prothétique comprenant un arrangement de fils définissant au moins deux faces pour ledit tissu, ledit tissu comprenant sur au moins une de ses faces un ou plusieurs picots saillant vers l'extérieur par rapport à ladite face : de tels tissus peuvent être préparés par exemple comme décrit dans WO01/81667.

Des tissus avec picots convenant à la présente invention sont également disponibles commercialement auprès de la société Sofradim Production sous la dénomination commerciale Parietex® Progrip ou encore Parietene® Progrip.

La composition comprenant le matériau hydrosoluble peut être préparée sous la forme d'une solution dans l'eau, si nécessaire en chauffant la solution jusqu'au point de fusion du matériau utilisé. Alternativement, la composition peut être préparée par chauffage du matériau seul à une température supérieure ou égale à la température de fusion dudit matériau, de manière à obtenir une composition liquide ou visqueuse.

La composition comprenant le matériau hydrosoluble à l'état liquide présente de préférence une viscosité permettant de la prélever à l'aide d'un pinceau ou d'un rouleau. La composition est ensuite appliquée sur les picots, par exemple avec un pinceau ou avec un rouleau. Pour une répartition la plus homogène possible de la composition sur les picots, il est préférable d'utiliser un rouleau. Il est également possible de faire varier la viscosité de la composition de matériau, soit en adaptant la température soit en adaptant la concentration de matériau dans la composition, selon que l'on souhaite obtenir un matériau qui se dissout rapidement ou non au contact de l'eau et/ou des fluides biologiques après séchage.

Une fois la composition comprenant le matériau appliquée sur les picots, on laisse sécher et/ou refroidir. En particulier, lorsque la composition est une solution du matériau hydrosoluble dans l'eau, on laisse sécher la composition afin que l'eau s'évapore et qu'il ne subsiste au final sur les picots essentiellement que du matériau hydrosoluble. Si on a chauffé la composition lors de la solubilisation du matériau hydrosoluble, on laisse également refroidir la composition. Lorsque la composition consiste en le matériau hydrosoluble seul chauffé à l'état liquide, on laisse refroidir la composition jusqu'à une température inférieure à la température de fusion dudit matériau. Pendant le séchage et/ou le refroidissement tels que décrits ci-dessus, la composition de matériau hydrosoluble répandue sur les picots se fige et enrobe la tête des picots. Ainsi, la tête des picots présente une surface particulièrement lisse. Lorsque deux picots viennent en contact l'un avec l'autre, ils glissent l'un sur l'autre sans s'opposer de résistance.

Ainsi, lorsque le chirurgien souhaite implanter une prothèse formée à partir d'un tissu selon l'invention, il peut facilement enrouler cette prothèse sur elle-même en repliant la face munie des picots recouverts vers l'intérieur ou vers l'extérieur. Ainsi, les picots sont protégés du frottement contre les parois du trocart ou contre tout autre élément extérieur de l'environnement.

Une fois la prothèse acheminée jusqu'au site d'implantation via le trocart, la prothèse peut être déroulée et déployée aisément car les picots, recouverts du matériau hydrosoluble encore sous forme figée, glissent les uns sur les autres et ne s'opposent pas de résistance. Ainsi, même si les picots ont été entremêlés lors de l'enroulage de la prothèse, leur démêlage s'effectue aisément.

La prothèse est déployée et les picots entrent peu à peu en contact avec les fluides biologiques au sein desquels ils se solubilisent progressivement. Durant cette solubilisation progressive, qui peut durer de quelques secondes à quelques minutes, le chirurgien peut encore mouvoir la prothèse aisément afin de la positionner correctement vis-à-vis du défaut herniaire à combler par exemple, ou vis-à-vis d'un autre tissu prothétique présent auquel le chirurgien souhaite fixer la prothèse.

Une fois que l'ensemble du matériau hydrosoluble présent sur les picots du tissu de la prothèse a été dissous dans les fluides biologiques, éventuellement avec l'aide d'une solution saline que le chirurgien ajoute pour accélérer la solubilisation du matériau hydrosoluble, les picots recouvrent leurs propriétés d'accroche, dues par exemple à la nature du matériau les constituant ainsi qu'à leur forme en crochet. La prothèse peut alors être fixée, soit à un autre tissu, soit à une paroi biologique, les picots n'ayant subi aucun dommage durant le transport de la prothèse dans le conduit du trocart.

Les exemples suivants illustrent l'invention.

### EXEMPLE 1 :

On dispose d'un tissu prothétique de taille 15X10 cm² comprenant des picots tel que décrit dans WO01/81667. Les picots sont réalisés à partir d'un fil monofilament en acide polylactique (PLA).

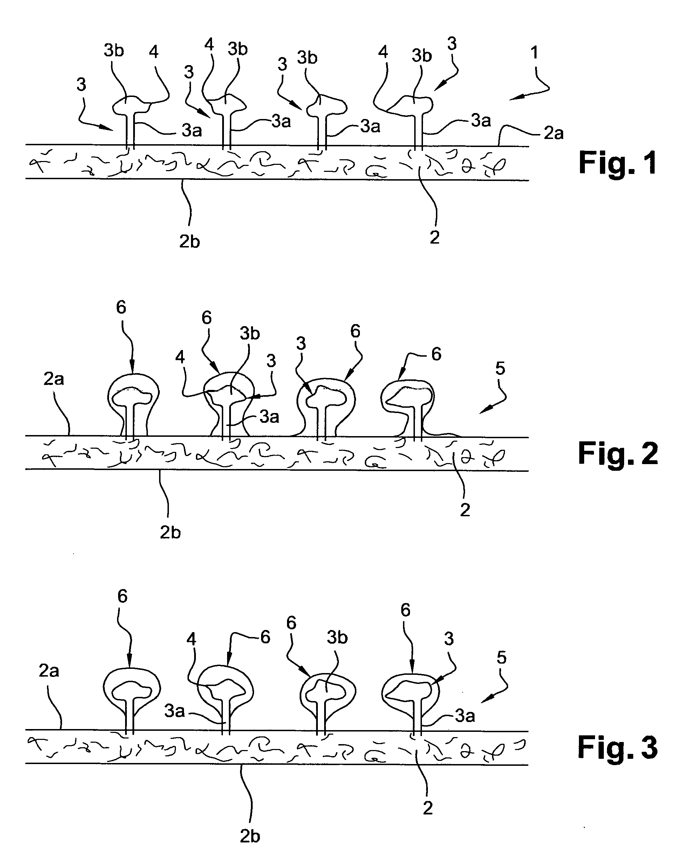

Une représentation schématique d'une coupe d'un tel tissu est donnée à la figure 1 : le tissu 1 est formé d'un arrangement 2 de fils définissant deux faces 2a et 2b opposées. Le tissu 1 comprend sur sa face 2a des picots 3 saillant de cette face. Chaque picot 3 est pourvu d'une tige 3a et d'une tête 3b. Comme il apparaît sur cette figure 1, les têtes 3a des picots présente des aspérités 4 contribuant aux propriétés d'accroche des picots.

5 g de polyéthylène glycol de masse molaire 4000 (« PEG 4000 » de la société FLUKA) dont la température de fusion est comprise entre 53°C et 59°C, a été chauffé à 60°C de façon à obtenir un liquide homogène. Les picots du tissu prothétique ont été enrobés avec le PEG 4000 à l'état liquide, en utilisant un pinceau ou par immersion des picots dans la composition de PEG 4000 liquide.

Par exemple, si on utilise un pinceau, et en fonction de la viscosité de la composition de PEG 4000, l'enrobage peut être réalisé par plusieurs passages successifs de pinceaux sur les picots.

On a ensuite laissé refroidir le tissu ainsi recouvert à température ambiante (environ 20°C). La composition de PEG 4000 s'est figée et a enrobé les picots les recouvrant d'un revêtement solide lisse.

Une représentation schématique d'une coupe d'un tel tissu 5 selon l'invention est donnée à la figure 2 : le tissu prothétique 5 selon l'invention comporte des picots 3 revêtus d'un matériau hydrosoluble, sous la forme d'une couche 6 solide dans l'exemple représenté. La couche 6 solide de matériau hydrosoluble, à savoir du polyéthylène glycol de poids moléculaire 4000 dans le présent exemple, enrobe totalement la tête 3b de chaque picot 3 ainsi qu'une partie de la tige 3a. Comme il apparaît clairement sur la figure 2, la surface de la couche 6 est lisse et les têtes 3b des picots 3 ne présentent plus d'aspérités 4 accessibles à tout autre tissu.

Ainsi, l'enrobage des picots par du PEG 4000 diminue l'accrochage du tissu et facilite sa manipulation. Dans les conditions utilisées, la masse de PEG 4000 ajoutée correspond à environ 50% de la masse du tissu de départ. Le tissu avec les picots enrobés a ensuite été lavé dans de l'eau à 37°C : les picots ont retrouvé leurs propriétés d'accrochage.

Il est possible de fabriquer des prothèses pour le traitement des hernies à partir du tissu avec picots revêtus de polyéthylène glycol tel que décrit dans le présent exemple, en découpant dans ledit tissu une forme rectangulaire ou ronde, ou une forme adaptée à l'anatomie de l'organe à traiter.

Le tissu avec picots revêtus de polyéthylène glycol tel que décrit dans le présent exemple est particulièrement adapté à la fabrication de prothèses pour le traitement de hernie par voie coelioscopique. En effet, de telles prothèses peuvent être enroulées sur elles-mêmes, picots à l'intérieur et/ou à l'extérieur et être acheminées ainsi dans le conduit d'un trocart jusqu'au site d'implantation sans risque d'endommagement des picots. Par ailleurs, du fait de leur revêtement en polyéthylène glycol hydrosoluble, les picots ne font pas obstacle au déploiement dans un premier temps de la prothèse une fois celle-ci libérée du trocart sur le site d'implantation : au bout de quelques secondes, après solubilisation due polyéthylène glycol par les fluides biologiques, les picots retrouvent leurs propriétés d'accrochage et peuvent servir à fixer la prothèse de la façon souhaitée par le chirurgien.

### EXEMPLE 2 :

On dispose du même tissu prothétique de départ que dans l'EXEMPLE 1 ci-dessus et représenté à la figure 1.

Quatre mélanges ont été préparés avec des proportions différentes de polyéthylène glycol de masse molaire 4000 (« PEG 4000 » de la société FLUKA) et de polyéthylène glycol de masse molaire 1000 (« PEG 1000 » de la société FLUKA, dont la température de fusion est comprise entre 33°C et 39°C) comme présenté dans le tableau 1 ci-dessous. Ces mélanges ont été chauffés à 60°C de façon à obtenir un liquide homogène. Les picots d'échantillons du tissu prothétique ont été enrobés avec lesdits mélanges en utilisant un pinceau.

**Tableau 1 : Proportions de PEG 1000 et PEG 4000 des différents mélanges (proportions données en poids).**

| Mélange | Proportion PEG 1000/PEG 4000 |
|---|---|
| N° 1 | 10/90 |
| N° 2 | 30/70 |
| N° 3 | 25/75 |
| N° 4 | 50/50 |

On obtient un tissu prothétique 5 selon l'invention tel que représenté à la figure 2.

L'enrobage des picots par les mélanges de polyéthylène glycol diminue l'accrochage du tissu et facilite sa manipulation. Dans les conditions utilisées, la masse de mélange ajouté varie de 30 à 100 % par rapport à la masse du tissu de départ. Ces données sont présentées sur le tableau 2.

**Tableau 2 : Masse de tissu et de mélange des différents échantillons (appelés Grip 1 à 9).**

| Echantillon | masse tissu (mg) | Mélange | Masse Mélange (mg) | Rapport masse mélange (mg)/masse tissu (mg) |
|---|---|---|---|---|
| Grip 1 | 255 | N°1 | 303 | 1,19 |
| Grip 2 | 519 | N°1 | 388 | 0,75 |
| Grip 3 | 397 | N°1 | 212 | 0,53 |
| Grip 4 | 713 | N°4 | 434 | 0,61 |
| Grip 5 | 638 | N°4 | 354 | 0,55 |
| Grip 6 | 613 | N°3 | 468 | 0,76 |
| Grip 7 | 600 | N°3 | 367 | 0,61 |
| Grip 8 | 627 | N°2 | 376 | 0,60 |
| Grip 9 | 776 | N°2 | 216 | 0,28 |

### Tests de solubilisation des mélanges :

Afin d'évaluer le temps nécessaire pour solubiliser les mélanges ajoutés sur les picots du tissu prothétique, les échantillons préparés comme décrit ci-dessus ont été introduits dans de l'eau à 37°C pendant quelques secondes, jusqu'à ce que les picots retrouvent leur pouvoir d'accrochage.

Ces tests ont montré que les mélanges se solubilisent rapidement (Tableau 3 ci-dessous), et que le temps nécessaire pour retrouver les propriétés d'accrochage des picots est de l'ordre de 20 secondes.

**Tableau 3 : Temps de solubilisation et masses des mélanges après les tests de solubilisation dans l'eau (N/A : non applicable)**

| Echantillon | Temps de dissolution dans de l'eau à 37 °C (s) | Masse de Mélange restant après solubilisation (mg) | % de Mélange restant |
|---|---|---|---|
| Grip 1 | N/A | 1 | 0,33 |
| Grip 2 | 10 | 91 | 23,45 |
| Grip 3 | 20 | 48 | 22,64 |
| Grip 4 | 10 | 73 | 16,82 |
| Grip 5 | 20 | 30 | 8,47 |
| Grip 6 | 18 | 59 | 12,85 |
| Grip 7 | 10 | 63 | 18,00 |
| Grip 8 | 18 | 5 | 1,33 |
| Grip 9 | 10 | 32 | 16,49 |

### EXEMPLE 3 :

On dispose du même tissu prothétique de départ que dans l'EXEMPLE 1 ci-dessus, décrit à la figure 1.

Sept mélanges ont été préparés avec des proportions différentes de polyéthylène glycol de masse molaire 4000 (« PEG 4000 » de la société FLUKA), de polyéthylène glycol de masse molaire 2000 (« PEG 2000 » de la société FLUKA) et de polyéthylène glycol de masse molaire 1000 (« PEG 1000 » de la société FLUKA) comme présenté dans le tableau 4 ci-dessous. Ces mélanges ont été chauffés à 60°C de façon à obtenir un liquide homogène. Les picots des échantillons du tissu ont été enrobés avec les mélanges en utilisant un pinceau.

**Tableau 4 : Proportions de PEG 1000, PEG 2000 et PEG 4000 des différents mélanges (proportions données en poids).**

| Mélange | PEG 1000/PEG 2000 | PEG 1000 / PEG 4000 |
|---|---|---|
| N° 1 | | 10 / 90 |
| N° 2 | | 30 / 70 |
| N° 3 | | 25 / 75 |
| N° 4 | | 50 / 50 |
| N°5 | 0/100 | |
| N°6 | 50/50 | |
| N°7 | 30/70 | |

On obtient un tissu prothétique 5 selon l'invention tel que représenté à la figure 2.

L'enrobage des picots par les mélanges décrits dans le tableau 4 diminue l'accrochage du tissu et facilite sa manipulation. Dans les conditions utilisées, la masse de mélange ajouté varie de 20 à 110 % par rapport à la masse du tissu de départ, ces données sont présentées sur le tableau 5 ci-dessous.

**Tableau 5 : Masse de tissu et de mélange des différents échantillons (appelés Grip 10 à 28).**

| Echantillon | masse tissu (mg) | Mélange | masse Mélange (mg) | Rapport masse mélange (mg)/masse tissu (mg) |
|---|---|---|---|---|
| Grip 10 | 650 | N°5 | 651 | 1,00 |
| Grip 11 | 821 | N°2 | 472 | 0,57 |
| Grip 12 | 698 | N°4 | 242 | 0,35 |
| Grip 13 | 610 | N°5 | 142 | 0,23 |
| Grip 14 | 490 | N°2 | 264 | 0,54 |
| Grip 15 | 474 | N°2 | 527 | 1,10 |
| Grip 16 | 554 | N°5 | 427 | 0,77 |
| Grip 17 | 637 | N°5 | 458 | 0,72 |
| Grip 18 | 609 | N°4 | 477 | 0,78 |
| Grip 19 | 575 | N°4 | 165 | 0,29 |
| Grip 20 | 561 | N°6 | 314 | 0,56 |
| Grip 21 | 450 | N°2 | 228 | 0,51 |
| Grip 22 | 637 | N°6 | 229 | 0,36 |
| Grip 23 | 526 | N°2 | 107 | 0,20 |
| Grip 24 | 529 | N°6 | 390 | 0,74 |
| Grip 25 | 634 | N°7 | 434 | 0,68 |
| Grip 26 | 624 | N°6 | 200 | 0,32 |
| Grip 27 | 598 | N°7 | 229 | 0,38 |
| Grip 28 | 665 | N°7 | 254 | 0,38 |

### Tests de solubilisation des mélanges :

Afin d'évaluer le temps nécessaire pour solubiliser les mélanges ajoutés sur les picots du tissu, les échantillons préparés comme décrit ci-dessus ont été maintenus sur des lingettes humidifiées à avec de l'eau à 40°C pendant plusieurs temps prédéfinis (10, 15 et 20 secondes). A l'issue des temps mentionnés précédemment, les propriétés d'accrochages des picots ont été évaluées ainsi que la masse de mélange résiduel sur les échantillons.

Ces tests ont montré que le mélange se solubilise rapidement (voir Tableaux 6 à 8 ci-dessous), et que le temps nécessaire pour retrouver les propriétés d'accrochage des picots est de l'ordre de 20 secondes.

**Tableau 6 : Masse du mélange après les tests de solubilisation sur des lingettes humidifiées avec de l'eau à 40°C, en fonction du temps de solubilisation (N/A : non applicable)**

| Echantillon | Masse mélange à 10s (mg) | Masse mélange à 15s (mg) | Masse mélange à 20s (mg) | Masse mélange à 30s (mg) |
|---|---|---|---|---|
| Grip 15 | N/A | N/A | 304 | 252 |
| Grip 17 | 282 | N/A | 184 | N/A |
| Grip 18 | 396 | N/A | 335 | N/A |
| Grip 21 | 106 | N/A | 84 | N/A |
| Grip 22 | 101 | N/A | 47 | N/A |
| Grip 25 | N/A | 128 | 110 | N/A |

**Tableau 7 : Rapport Masse du mélange (mg)/masse du tissu (mg) après les tests de solubilisation sur des lingettes humidifiées avec de l'eau à 40°C, en fonction du temps de solubilisation (N/A : non applicable)**

| Echantillon | Rapport masse Mélange (mg)/masse du tissu (mg) à 10s | Rapport masse Mélange (mg)/masse du tissu (mg) à 15s | Rapport masse Mélange (mg)/masse du tissu (mg) à 20s | Rapport masse Mélange (mg)/masse du tissu (mg) à 30s |
|---|---|---|---|---|
| Grip 15 | N/A | N/A | 0,64 | 0,53 |
| Grip 17 | 0,44 | N/A | 0,29 | N/A |
| Grip 18 | 0,65 | N/A | 0,55 | N/A |
| Grip 21 | 0,23 | N/A | 0,18 | N/A |
| Grip 22 | 0,16 | N/A | 0,07 | N/A |
| Grip 25 | N/A | 0,20 | 0,17 | N/A |

**Tableau 8 : % du mélange restant après les tests de solubilisation sur des lingettes humidifiées avec de l'eau à 40°C, en fonction du temps de solubilisation (N/A : non applicable)**

| Echantillon | % Mélange à 10s | % Mélange à 15s | % Mélange à 20s | % Mélange à 30s |
|---|---|---|---|---|
| Grip 15 | N/A | N/A | 57,7 | 47,8 |
| Grip 17 | 61,6 | N/A | 40,2 | N/A |
| Grip 18 | 83,0 | N/A | 70,2 | N/A |
| Grip 21 | 46,5 | N/A | 36,8 | N/A |
| Grip 22 | 44,1 | N/A | 20,5 | N/A |
| Grip 25 | N/A | 29,5 | 25,3 | N/A |

Les tableaux 6 à 8 montrent que la quantité de mélange déposée au départ joue un rôle important sur sa solubilisation : pour les échantillons ayant une petite quantité de mélange, presque la totalité de mélange déposé se trouve sur les picots du tissu, qui sont directement en contact avec les lingettes humides au moment de la solubilisation.

Il est également à observer que l'ajout du PEG 1000 facilite la solubilisation. La quantité résiduelle de PEG sera plus faible, et dans un délai plus court.

### Stabilité de l'enduction :

Etant donné la faible température de fusion du PEG 1000 (33 à 39°C) quelques tests ont été réalisés afin d'évaluer la stabilité de l'enduction à 40°C. Après 24 heures dans une étuve à 40°C, un échantillon enduit seulement avec du PEG 1000, montre que dans ces conditions, le PEG 1000 fond. Par contre le tissu enduit avec des mélanges de PEG 1000/PEG 4000 ou encore PEG1000/PEG 2000 ne présente aucune modification de l'induction dans ces mêmes conditions.

L'ajout du PEG 1000 présente un avantage pour une solubilisation rapide du mélange de PEG.

### EXEMPLE 4 :

On dispose du même tissu prothétique de départ que dans l'EXEMPLE 1 ci-dessus, décrit à la figure 1..

Quatre mélanges ont été préparés avec des proportions différentes de polyéthylène glycol de masse molaire 2000 (« PEG 2000 » de la société FLUKA) et de polyéthylène glycol de masse molaire 1000 (« PEG 1000 » de la société FLUKA) comme présenté dans le tableau 9 ci-dessous. Ces mélanges ont été chauffés à 60°C de façon à obtenir un liquide homogène. Les picots des échantillons du tissu ont été enrobés avec les mélanges en utilisant un rouleau en mousse.

**Tableau 9 : Proportions de PEG 1000, PEG 2000 et PEG 4000 données en poids/poids**

| Mélange | PEG 1000/PEG 2000 |
|---|---|
| N°5 | 0/100 |
| N°6 | 50/50 |
| N°7 | 30/70 |
| N°8 | 100/0 |

On obtient un tissu prothétique 5 selon l'invention tel que représenté à la figure 3. Comme il apparaît sur cette figure, la couche 6 solide de matériau hydrosoluble, à savoir le mélange de polyéthylène glycol du présent exemple, enrobe précisément la tête 3b de chaque picot 3. Les tiges 3a des picots restent non enrobées.

Ainsi, l'enrobage des picots par les mélanges du présent exemple, en utilisant un rouleau, est très efficace. Les échantillons ainsi obtenus sont très homogènes et la déposition est bien dirigée sur les têtes des picots du tissu. Dans les conditions utilisées la masse de mélange ajouté varie de 38 à 55 % par rapport à la masse du tissu de départ. Ces données sont présentées dans le tableau 10 ci-dessous.

Du fait de l'enrobage dirigé sur les têtes des picots, on obtient à la fois une manipulation aisée du tissu selon l'invention, comme décrit dans les précédents exemples, et à la fois une solubilisation facilitée et plus rapide lorsque le tissu est mis au contact des fluides biologiques.

**Tableau 10 : Masse de tissu et de mélange des différents échantillons**

| Echantillon | masse tissu (mg) | Mélange | masse Mélange (mg) | Rapport masse mélange (mg)/masse tissu (mg) |
|---|---|---|---|---|
| Grip 29 | 889 | N° 6 | 442 | 0,50 |
| Grip 30 | 756 | N° 6 | 415 | 0,55 |
| Grip 31 | 676 | N° 7 | 262 | 0,39 |
| Grip 32 | 634 | N° 5 | 239 | 0,38 |
| Grip 33 | 788 | N° 8 | 402 | 0,51 |

Les tissus selon l'invention avec picots revêtus de polyéthylène glycol tels que décrits dans les exemples 1 à 4 ci-dessus sont particulièrement adaptés à la fabrication de prothèses pour le traitement de hernie par voie coelioscopique. En effet, de telles prothèses peuvent être enroulées sur elles-mêmes, picots à l'intérieur et/ou à l'extérieur et être acheminées ainsi dans le conduit d'un trocart jusqu'au site d'implantation sans risque d'endommagement des picots. Par ailleurs, du fait de leur revêtement en polyéthylène glycol hydrosoluble, les picots ne font pas obstacle au déploiement dans un premier temps de la prothèse une fois celle-ci libérée du trocart sur le site d'implantation : au bout de quelques secondes, les picots retrouvent leurs propriétés d'accrochage et peuvent servir à fixer la prothèse de la façon souhaitée par le chirurgien.

## Revendications

1. Tissu prothétique (5) comprenant un arrangement (2) de fils définissant au moins deux faces (2a, 2b) pour ledit tissu, ledit tissu comprenant sur au moins une de ses faces un ou plusieurs picots (3) saillant vers l'extérieur par rapport à ladite face, **caractérisé en ce que** lesdits picots sont recouverts d'un revêtement (6) en matériau biocompatible hydrosoluble.

2. Tissu (5) selon la revendication 1, **caractérisé en ce que** ledit matériau hydrosoluble est biodégradable.

3. Tissu (5) selon la revendication 1 ou 2, **caractérisé en ce que** ledit matériau hydrosoluble est choisi parmi les polyéthylène glycols (PEG), les acétates de polyvinyle (PVA), la gélatine, l'acide polyglucoronique, l'acide hyaluronique, la carboxyméthycellulose, les éthers de cellulose, les chitosan et leurs mélanges.

4. Tissu (5) selon l'une quelconque des revendications précédentes, **caractérisé en ce que** ledit matériau hydrosoluble est un polyéthylène glycol de masse molaire inférieure ou égale à 40 000 Da, de préférence inférieure ou égale à 20 000 Da.

5. Tissu (5) selon la revendication 5, **caractérisé en ce que** ladite masse molaire varie de 1000 à 20 000 Da.

6. Tissu (5) selon l'une quelconque des revendications précédentes, **caractérisé en ce que** ledit matériau hydrosoluble comprend au moins un polyéthylène glycol de masse molaire environ 1000 Da.

7. Tissu (5) selon la revendication 6, **caractérisé en ce que** ledit matériau hydrosoluble comprend en outre un polyéthylène glycol de masse molaire différente de 1000 Da.

8. Procédé pour recouvrir un tissu prothétique (1) comprenant un arrangement (2) de fils définissant au moins deux faces (2a, 2b) pour ledit tissu, ledit tissu comprenant sur au moins une de ses faces un ou plusieurs picots (3) saillant vers l'extérieur par rapport à ladite face, **caractérisé en ce qu'**il comprend les étapes suivantes :
- a°) on prépare une composition comprenant au moins un matériau biocompatible hydrosoluble à l'état liquide,
- b°) on applique une couche de ladite composition sur lesdits picots.

9. Procédé selon la revendication 8, **caractérisé en ce que** l'étape b°) est réalisée à l'aide d'un rouleau.

10. Procédé selon la revendication 8 ou 9, **caractérisé en ce que** ladite composition est obtenue par solubilisation dudit matériau hydrosoluble dans de l'eau, éventuellement en chauffant ladite composition à la température de fusion dudit matériau hydrosoluble.

11. Procédé selon la revendication 8 ou 9, **caractérisé en ce que** ladite composition est obtenue par chauffage dudit matériau hydrosoluble seul, à une température supérieure à la température de fusion dudit matériau hydrosoluble.

12. Prothèse pour le traitement de hernie, **caractérisée en ce qu'**elle est fabriquée à partir d'un tissu (5) selon l'une quelconque des revendications 1 à 7 ou à partir d'un tissu obtenu par le procédé selon l'une quelconque des revendications 8 à 11.

## Patentansprüche

1. Prothetisches Gewebe (5), umfassend eine Anordnung (2) von Garnen, die mindestens zweit Seiten (2a, 2b) für das Gewebe definieren, wobei das Gewebe auf mindestens einer seiner Seiten einen oder mehrere Widerhaken (3) umfasst, die mit Bezug auf die Seite nach außen hervorstehen, **dadurch gekennzeichnet, dass** die Widerhaken mit einer Beschichtung (6) aus einem biokompatiblen wasserlöslichen Material hergestellt sind.

2. Gewebe (5) nach Anspruch 1, **dadurch gekennzeichnet, dass** das wasserlösliche Material biologisch abbaubar ist.

3. Gewebe (5) nach Anspruch 1 oder 2, **dadurch gekennzeichnet, dass** das wasserlösliche Material ausgewählt ist aus Polyethylenglykolen (PEG),Polyvinylacetaten (PVA), Gelatine, Polyglucuronsäure, Hyaluronsäure, Carboxymethylcellulose, Zelluloseethern, Chitosan und ihren Mischungen.

4. Gewebe (5) nach einem der vorhergehenden Ansprüche, **dadurch gekennzeichnet, dass** das wasserlösliche Material ein Polyethylenglykol mit einer molaren Masse von weniger als oder gleich 40000 Da, vorzugsweise von weniger als oder gleich 20000 Da ist.

5. Gewebe (5) nach Anspruch 5, **dadurch gekennzeichnet, dass** die molare Masse von 1000 bis 20000 Da variiert.

6. Gewebe (5) nach einem der vorhergehenden Ansprüche, **dadurch gekennzeichnet, dass** das wasserlösliche Material mindestens ein Polyethylenglykol mit einer molaren Masse von ungefähr 1000 Da umfasst.

7. Gewebe (5) nach Anspruch 6, **dadurch gekennzeichnet, dass** das wasserlösliche Material außerdem ein Polyethylenglykol mit einer molaren Masse aufweist, die verschiedenen von 1000 Da ist.

8. Verfahren zum Beschichten eines prothetischen Gewebes (1), umfassend eine Anordnung (2) von Garnen, die mindestens zwei Seiten (2a, 2b) für das Gewebe definieren, wobei das Gewebe auf mindestens einer seiner Seiten einen oder mehrere Widerhaken (3) umfasst, die mit Bezug auf die Seite nach außen hervorstehen, **dadurch gekennzeichnet, dass** es die folgenden Schritte umfasst:
a) es wird eine Zusammensetzung zubereitet, umfassend mindestens ein biokompatibles wasserlösliches Material im flüssigen Zustand,
b) es wird eine Schicht der Zusammensetzung auf die Widerhaken aufgetragen.

9. Verfahren nach Anspruch 8, **dadurch gekennzeichnet, dass** der Schritt b) mit Hilfe einer Rolle durchgeführt wird.

10. Verfahren nach Anspruch 8 oder 9, **dadurch gekennzeichnet, dass** die Zusammensetzung durch die Solubilisierung des wasserlöslichen Materials in Wasser erhalten wird, eventuell indem die Zusammensetzung auf die Fusionstemperatur des wasserlöslichen Materials erhitzt wird.

11. Verfahren nach Anspruch 8 oder 9, **dadurch gekennzeichnet, dass** die Zusammensetzung durch Erhitzung des wasserlöslichen Materials allein auf eine höhere Temperatur als die Fusionstemperatur des wasserlöslichen Materials erhalten wird.

12. Prothese zur Behandlung von Hernien, **dadurch gekennzeichnet, dass** sie aus einem Gewebe (5) nach einem der Ansprüche 1 bis 7 oder aus einem Gewebe, erhalten durch das Verfahren nach einem der Ansprüche 8 bis 11 hergestellt wird.

## Claims

1. Prosthetic fabric (5) comprising an arrangement (2) of yarns defining at least two faces (2a, 2b) for said fabric, said fabric comprising on at least one of its faces, one or more barbs (3) that protrude outwards relative to said face, **characterized in that** said barbs are covered with a coating made of a water-soluble biocompatible material.

2. Fabric (5) according to Claim 1, **characterized in that** said water-soluble material is biodegradable.

3. Fabric (5) according to Claim 1 or 2, **characterized in that** said water-soluble material is chosen from polyethylene glycols (PEGs), polyvinyl acetates (PVAs), gelatin, polyglucuronic acid, hyaluronic acid, carboxymethyl cellulose, cellulose ethers, chitosans and mixtures thereof.

4. Fabric (5) according to any one of the preceding claims, **characterized in that** said water-soluble material is a polyethylene glycol having a molar mass of less than or equal to 40 000 Da, preferably less than or equal to 20 000 Da.

5. Fabric (5) according to Claim 5, **characterized in that** said molar mass varies from 1000 to 20 000 Da.

6. Fabric (5) according to any one of the preceding claims, **characterized in that** said water-soluble material comprises at least one polyethylene glycol having a molar mass of around 1000 Da.

7. Fabric (5) according to Claim 6, **characterized in that** said water-soluble material also comprises a polyethylene glycol having a molar mass different from 1000 Da.

8. Process for covering a prosthetic fabric (1) comprising an arrangement (2) of yarns defining at least two faces (2a, 2b) for said fabric, said fabric comprising on at least one of its faces, one or more barbs (3) that protrude outwards relative to said face, **characterized in that** it comprises the following steps:
- a°) a composition is prepared that comprises at least one water-soluble biocompatible material in the liquid state; and
- b°) a layer of said composition is applied to said barbs.

9. Process according to Claim 8, **characterized in that** step b°) is carried out using a roll.

10. Process according to Claim 8 or 9, **characterized in that** said composition is obtained by solubilization of said water-soluble material in water, optionally by heating said composition to the melting point of said water-soluble material.

11. Process according to Claim 8 or 9, **characterized in that** said composition is obtained by heating said water-soluble material alone, to a temperature above the melting point of said water-soluble material.

12. Prosthesis for the treatment of hernias, **characterized in that** it is manufactured from a fabric (5) according to any one of Claims 1 to 7 or from a fabric obtained by the process according to one of Claims 8 to 11.
